# EUROPÄISCHE PATENTSCHRIFT

(11) **EP 0 422 534 B1**
(45) Veröffentlichungstag und Bekanntmachung des Hinweises auf die Patenterteilung: **19.01.1994**
(21) Anmeldenummer: 90119204.7
(22) Anmeldetag: 06.10.1990
(51) Int. Cl.: C07C 209/60

(54) **Verfahren zur Herstellung von N-Alkyl-und N,N-Dialkyl-diaminoethanen**
Process for the preparation of N-alkyl and N,N-dialkyl-diamino ethanes
Procédé pour préparation de N-alkyl et de N,N-dialkyl-diaminoéthanes

(30) Priorität: 13.10.1989 DE 3934191
(43) Veröffentlichungstag der Anmeldung: 17.04.1991
(73) Patentinhaber: BASF Aktiengesellschaft, 67063 Ludwigshafen (DE)
(72) Erfinder: Hohmann, Andreas, Dr., W-6700 Ludwigshafen (DE); Reuther, Wolfgang, Dr., W-6900 Heidelberg (DE); Bochnitschek, Werner, Dr., W-6700 Ludwigshafen (DE)

(56) Entgegenhaltungen:
- US-A- 2 318 729
- BULLETIN OF THE ACADEMY OF SCIENCES OF THE USSR, DIVISION OF CHEMICAL SCIENCE, Band 36, April 1987, Seiten 873-874, New York, US; R.K. ALEKPEROV et al.: "N-beta-dimethylaminoethylurethylane as a chemical mutagen with possible carbomoylating action"
- CHEMICAL ABSTRACTS, Band 99, 1983, Seite 564, Zusammenfassung Nr. 157813j, Columbus, Ohio, US; & JP-A-58 46 042
- CHEMICAL ABSTRACTS, Band 109, Nr. 25, 19. Dezember 1988, Seite 791, Zusammenfassung Nr. 230269t,230270m, Columbus, Ohio, US; & JP-A-63122652, & JP-A-63122653

## Beschreibung

Die vorliegende Erfindung betrifft ein neues und verbessertes Verfahren zur Herstellung von unsymmetrisch N-substituierten Diaminoethanen durch Umsetzung von Iminen mit Aminen in Gegenwart von Kohlendioxid.

Es ist bekannt, daß N-Alkyl- und N,N-Dialkyl-diaminoethane, z.B. durch Aminolyse von Phthalimiden mit anschließender Hydrolyse zum Ethylendiaminderivat (DD-A-17068), durch Aminolyse von 2-Chlorethylamin (Monatshefte der Chemie 84, 362 (1953), durch Reaktion von sek. Aminen mit Acrylamid mit nachfolgender Hoffmann-Umlagerung zum Diamin (Chem. Abstr. Vol. 96, 180762p sowie durch Umsetzung von Aminen, Formaldehyd und Blausäure zum N-substituierten Glycinnitril, das nach Hydrierung ebenfalls das gewünschte unsymmetrisch substituierte Ethylendiaminderivat ergibt, hergestellt werden können.

Die Aminoethylierung eines entsprechenden Amins mit Ethylenimin wurde sowohl mit (Izw. Akad. Nauk, SSSR, Ser. Khim. 1987, 4, 946-948) als auch ohne (US-A-2 318 729) saure Katalyse durchgeführt. Die Ausbeuten lassen zu wünschen übrig und die Reaktionszeiten sind lang.

In Chem. Abstr. Vol. 99, 157814 und Chem. Abstr. Vol. 99, 157813 werden zur Synthese von N-Methylethylendiamin und N,N-Dimethylethylendiamin Methylamin bzw. Dimethylamin und Ethylenimin mit 30 Mol.% bzw. 3 Mol.% Salzsäure als Katalysator verwendet. Das Amin/Ethyleniminverhältnis beträgt 5 : 1 (beim Methylamin) bzw. 3 : 1 (beim Dimethylamin). Eine Aufarbeitung ist nicht beschrieben, die gaschromatographisch ermittelten Ausbeuten liegen zwischen 80 und 90 %.

Das letztgenannte Verfahren ist allerdings auf die Verwendung eines Autoklaven angewiesen, da die leichtsiedenden Amine bei der vorgeschriebenen Reaktionstemperatur von 110 bis 140°C einen Druck von 8 bis 12 bar aufbauen. Weiter ist der Einsatz von Salzsäure wegen der damit verbundenen Korrosionsprobleme nachteilig. Da die zugesetzte Salzsäure im Rahmen der Aufarbeitung neutralisiert werden muß, um das Produkt vollständig freizusetzen, entstehen bei der beschriebenen Umsetzung z.T. beträchtliche Mengen an z.B. Natriumchlorid.

Der Erfindung lag nun die Aufgabe zugrunde, ein Verfahren zur Herstellung von unsymmetrisch substituierten Diaminoethanderivaten zu finden, das den vorbeschriebenen Nachteilen abhilft.

Demgemäß wurde ein neues und verbessertes Verfahren zur Herstellung von N-Alkyl- oder N,N-Dialkyl-1,2-diaminoethanen der allgemeinen Formel I 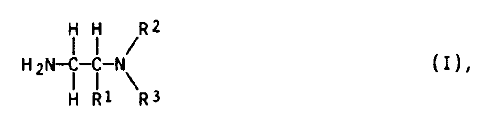
in der R¹ Wasserstoff oder Methyl, R² C₁- bis C₅-Alkyl und R³ Wasserstoff oder C₁- bis C₅-Alkyl bedeuten, mit der Maßgabe, daß die Summe der C-Atome der Reste R² und R³ für eine ganze Zahl des Bereichs 1 bis 6 steht, durch Umsetzung von Iminen der allgemeinen Formel II 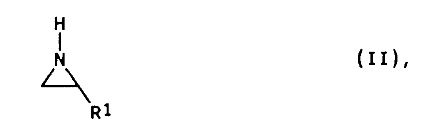
mit Aminen der allgemeinen Formel III 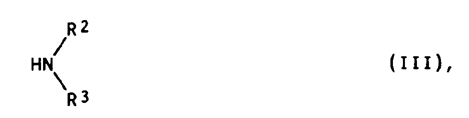
in denen R¹, R² und R³ die oben genannten Bedeutungen haben, gefunden, welches dadurch gekennzeichnet ist, daß man die Umsetzung in Gegenwart von Kohlendioxid bei Temperaturen von 10 bis 150°C und Drücken von 0,5 bis 50 bar, vorzugsweise bei 1 bis 10 bar, durchführt.

Die Verbindungen I sind nach folgender Methode zugänglich:

Zu dem umzusetzenden Amin (III), das sowohl mit als auch ohne Lösungsmittel vorgegeben werden kann, wird Kohlendioxid gegeben. Die Menge an CO₂ beträgt zwischen 0,1 und 2,0 Moläquivalenten, bezogen auf umzusetzendes Alkylenimin (II). Als Lösungsmittel eignen sich Wasser, Alkohole und alle unter den Reaktionsbedingungen inerten Flüssigkeiten.

Das Kohlendioxid kann sowohl in fester Form zugegeben als auch eingegast werden.

Zu der vorliegenden Reaktionslösung wird dann das Alkylenimin (II) gegeben. Das Molverhältnis Alkylenimin (II) zu Amin (III) liegt zwischen 1 : 1 und 0,1 : 1, bevorzugt zwischen 1 : 1 und 0,25 : 1. In vielen Fällen läßt sich das überschüssige Amin problemlos nach erfolgter Umsetzung wiedergewinnen. Wie das Amin kann auch das Alkylenimin lösungsmittelfrei oder als Lösung unterschiedlicher Konzentration eingesetzt werden. Der Lösungsmittelgehalt des Reaktionsansatzes kann zwischen 0 % und 90 % liegen.

Die Zugabezeit liegt zwischen 0,25 und 10 h. Bevorzugt ist der Bereich zwischen 2 und 4 h.

Die Reaktionstemperatur wird zwischen 10 °C und 150 °C gehalten. Sie wird nach oben durch die Rückflußtemperatur des Ansatzes begrenzt. Vorzugsweise bewegt man sich knapp unterhalb der Siedetemperatur des Reaktionsansatzes. Die Reaktionstemperatur kann während der Umsetzung variiert werden, aber auch konstant gehalten werden. Man arbeitet bei Drücken von 0,5 bis 50 bar, vorzugsweise bei 1 bis 10 bar, insbesondere bis 5 bar, besonders bevorzugt bei Atmosphärendruck.

Nach Zugabe des Alkylenimins wird 1 bis 10 h nachgerührt. Für die Temperaturführung gilt das oben gesagte.

Nach dem Ende der Umsetzung wird dem Reaktionsansatz Alkali- oder Erdalkalihydroxid zugegeben. Die eingesetzte Menge beträgt 1 bis 20, vorzugsweise 2 bis 4 Mol Hydroxid je Mol Kohlendioxid.

Der so vorbereitete Ansatz wird anschließend destillativ aufgearbeitet. Falls ein wäßriges System vorliegt, kann das Wasser auch im Vorwege durch Zugabe von Alkali- oder Erdalkalihydroxiden oder deren konzentrierten wäßrigen Lösungen entfernt werden und der entwässerte Ansatz zur Reinigung des Rohproduktes, falls dieses erforderlich ist, abschließend destilliert werden.

Zur Umsetzung mit Ethylenimin oder Propylenimin nach dem erfindungsgemäßen Verfahren eignen sich besonders Amine mit 1 bis 6 C-Atomen als Summe für R² und R³, wie Methylamin, Ethylamin, Propylamin, Dimethylamin, Methylethylamin, Methylpropylamin, Diethylamin, Ethylpropylamin und Dipropylamin.

Die N-Alkyl- und N,N-Dialkyl-1,2-aminoethane sind begehrte Zwischenprodukte für pharmazeutische Wirkstoffe.

### Herstellbeispiele

### Beispiel 1

In einem mit Rückflußkühler (Sole, < -10 °C), Rührer, Tropftrichter und Innenthermometer versehenen Kolben wurden 912 g 59,2 %ige wäßrige Dimethylaminlösung (12 mol) vorgelegt. In diese Lösung wurden anschließend 88 g (2 mol) Kohlendioxid eingegast. Nachdem alles Kohlendioxid eingegast war, wurden 287 g einer 60 %igen wäßrigen Ethyleniminlösung (4 mol) innerhalb von 2 Stunden gleichmäßig zugegeben. Der Ansatz wurde zwischen 38 °C und 40 °C gehalten.

Im Anschluß an die Ethyleniminzugabe wurde 6 Stunden bei 48 °C nachgerührt. Danach wurde für die Aufarbeitung die Solekühlung des Kühlers durch Kühlwasser ersetzt. Anschließend wurden 448 g 50 %ige Kalilauge in das Reaktionsgemisch eingerührt, der Ansatz nach beendeter Zugabe auf Rückflußtemperatur (ca. 107 °C) erhitzt und bei dieser Temperatur 0,25 h gehalten. Der Ansatz wurde auf 50 °C abgekühlt und die klare, farblose untere Phase abgetrennt. Im Anschluß wurde die obere Phase mit 300 g Ätznatron versetzt. Der Ansatz wurde 0,5 h bei 70 °C gerührt und nach erfolgter Phasentrennung die untere Phase abgetrennt.

Die obere Phase wurde in zwei Schritten mit je 15 g Ätznatron versetzt und 0.25 h bei 70 bis 80 °C gerührt. Anschließend wurde das Rohprodukt im Vakuum destilliert. In der Vorlage wurden 275 g farbloses Destillat aufgefangen.
- Ausbeute:: 275 g N,N-Dimethylaminoethylamin (78 %, bezogen auf Ethylenimin)
- Gehalt:: 98,6 % (GC)

- Aminzahl:: 22,40 mmol/g (99 % d. Th.)
- Wassergehalt:: 0,3 %
- tert. Stickstoff:: 11,19 mmol/g (98,4 % d. Th.)
- Brechungsindex (25 °C):: 1,4251 (Lit.: 1,4250 bei 25 °C)

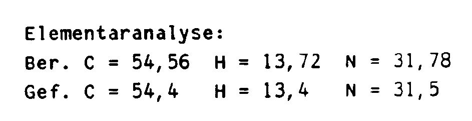

### Beispiel 2

In der unter Beispiel 1 beschriebenen Apparatur wurden 282 g 64 %ige wäßrige DMA-Lösung (4 mol) mit 66 g (1,5 mol) gasförmigem Kohlendioxid versetzt und innerhalb von 2 h 143 g 60 %ige wäßrige Ethyleniminlösung (2 mol) bei 60 bis 66 °C zugetropft, 1 h nachgerührt, 480 g 50 %ige Natronlauge zugetropft und danach 1 h bei 90 °C gerührt, wobei überschüssiges DMA abdampfte.

Nach Phasentrennung des zweiphasigen wurde die obere Phase Reaktionsansatzes wurde mit 73 g Ätznatron versetzt und bei 50 bis 60 °C im Vakuum destilliert.
- Ausbeute:: 74 g N,N-Dimethylaminoethylamin (42 %, bezogen auf Ethylenimin)
- Gehalt:: 99 %
- Wassergehalt:: 0,6 %

### Beispiel 3

In der unter Beispiel 1 beschriebenen Apparatur wurden 876 g 60 %ige wäßrige tert. Butylamin-Lösung (6 mol) mit 44 g (1 mol)gasförmigem Kohlendioxid versetzt und innerhalb von 2 h 143 g 60 %ige wäßrige Ethyleniminlösung (2 mol) bei 70 °C zugetropft, 5 h bei 70 °C nachgerührt, 320 g 50 %ige Natronlauge zugetropft und der Ansatz auf 110 °C erhitzt. Dabei destillierte ein tert.-Butylamin/Wasser-Gemisch über. Bei einer Übergangstemperatur von 100 °C wurde die Destillation beendet, die untere Phase des zweiphasigen Ansatzes abgetrennt und die obere Phase wie üblich mit 3 x 40 ml 50 %iger Natronlauge bei 90 °C entwässert.

Das GC des Rohproduktes (155 g) zeigte einen Gehalt von 77 % tert.-Butylaminoethylamin an.

### Beispiel 4

In der unter Beispiel 1 beschriebenen Apparatur wurden 730 g 60 %ige wäßrige Diethylaminlösung (6 mol) vorgelegt, mit 44 g Kohlendioxid (1 mol) versetzt und innerhalb von 2 h 143 g 60 %ige wäßrige Ethyleniminlösung (2 mol) bei einer Innentemperatur von 38 °C zugeführt.

Anschließend wurde 4 h bei 70 °C nachgerührt, der Ansatz mit 247 g 50 %iger Kalilauge versetzt, das überschüssige Diethylamin bis zu einer Innentemperatur von 98 °C abdestilliert, der zweiphasige Reaktionsansatz auf 50 °C abgekühlt und die untere Phase abgetrennt.

Nach Trocknung der Oberphase mit Ätznatron und nachfolgender Phasentrennung wurde das Rohprodukt über Ätznatron im Vakuum bei einer Übergangstemperatur von 45 bis 65 °C destilliert.
- Ausbeute:: 117 g N,N-Diethylaminoethylamin (50 %, bezogen auf Ethylenimin)
- Gehalt:: 97 % (GC)

### Beispiel 5

In der unter Beispiel 1 beschriebenen Apparatur wurden 383 g 71 %ige wäßrige Ethylaminlösung (6 mol) vorgelegt, mit 44 g Kohlendioxid (1 mol) versetzt und innerhalb von 2 h 143 g 60 %ige wäßrige Ethyleniminlösung (2 mol) bei einer Innentemperatur von 40 bis 50 °C zugefügt.

Danach wurde 6 h bei 55 °C nachgerührt, der Ansatz mit 244 g 50 %iger Kalilauge versetzt, auf eine Innentemperatur von 107 °C erhitzt und dabei das überschüssige Diethylamin entfernt. Der zweiphasige Reaktionsansatz wurde auf 50 °C abgekühlt und die untere Phase abgetrennt.

Die Oberphase wurde mit 105 g Ätznatron versetzt und anschließend die untere Phase abgetrennt. Das Rohprodukt (123 g, Wassergehalt 2 %) besaß einen Gehalt an Ethylaminoethylamin von 71 % (GC).

## Patentansprüche

1. Verfahren zur Herstellung von N-Alkyl- oder N,N-Dialkyl-1,2-diaminoethanen der allgemeinen Formel I 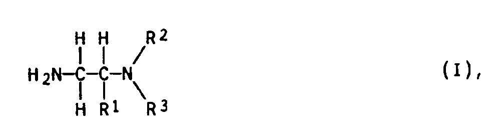 in der R¹ Wasserstoff oder Methyl, R² C₁- bis C₅-Alkyl und R³ Wasserstoff oder C₁- bis C₅-Alkyl bedeuten, mit der Maßgabe, daß die Summe der C-Atome der Reste R² und R³ für eine ganze Zahl des Bereichs 1 bis 6 steht, durch Umsetzung von Iminen der allgemeinen Formel II 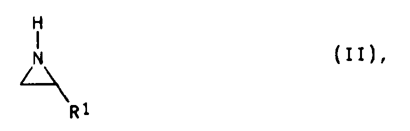 mit Aminen der allgemeinen Formel III 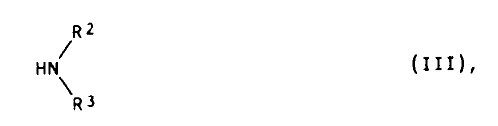 in denen R¹, R² und R³ die oben genannten Bedeutungen haben, dadurch gekennzeichnet, daß man die Umsetzung in Gegenwart von Kohlendioxid bei Temperaturen von 10 bis 150°C und Drücken von 0,5 bis 50 bar durchführt.

2. Verfahren nach Anspruch 1, dadurch gekennzeichnet, daß man das Imin II und das Amin III im Molverhältnis von 1:1 bis 0,1:1 einsetzt.

3. Verfahren nach Anspruch 1, dadurch gekennzeichnet, daß man das Imin II und das Amin III im Molverhältnis von 1:1 bis 0,25:1 einsetzt.

4. Verfahren nach Anspruch 1, dadurch gekennzeichnet, daß man 0,1 bis 2 Moläquivalent Kohlendioxid bezogen auf das eingesetzte Imin verwendet.

5. Verfahren nach Anspruch 1, dadurch gekennzeichnet, daß man die Umsetzung bei 1 bis 10 bar durchführt.

## Claims

1. A process for the preparation of an N-alkyl- or N,N-dialkyl-1,2-diaminoethane of the general formula I 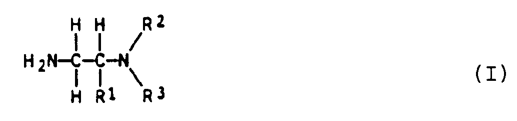 where R¹ is hydrogen or methyl, R² is C₁- to C₅-alkyl, and R³ is hydrogen or C₁- to C₅-alkyl, with the proviso that the total number of carbon atoms in R² and R³ is an integer from 1 to 6, by reacting an imine of the general formula II 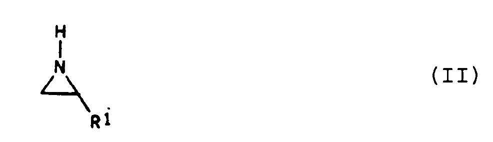 with an amine of the general formula III 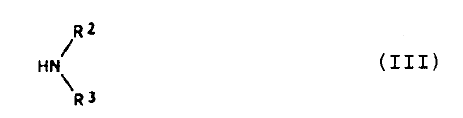 where R¹, R² and R³ are as defined above, which comprises carrying out the reaction in the presence of carbon dioxide at from 10 to 150°C and at from 0.5 to 50 bar.

2. A process as claimed in claim 1, wherein the imine II and the amine III are employed in a molar ratio of from 1:1 to 0.1:1.

3. A process as claimed in claim 1, wherein the imine II and the amine III are employed in a molar ratio of from 1:1 to 0.25:1.

4. A process as claimed in claim 1, wherein from 0.1 to 2 mol equivalents of carbon dioxide are used, based on the imine employed.

5. A process as claimed in claim 1, wherein the reaction is carried out at from 1 to 10 bar.

## Revendications

1. Procédé de préparation de N-alkyl- ou N,N-dialkyl-1,2-diaminoéthanes de la formule générale I 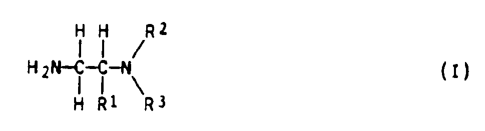 dans laquelle R¹ représente un atome d'hydrogène ou le radical méthyle, R² représente un radical alkyle en C₁ à C₅ et R³ représente un atome d'hydrogène ou un radical alkyle en C₁ à C₅, avec la condition que la somme des atomes de carbone des restes R² et R³ représente un nombre entier qui s'étend dans la plage de 1 à 6, par la réaction d'imines de la formule générale II 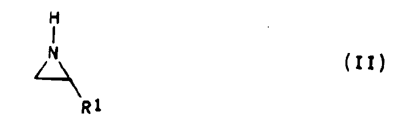 avec des amines de la formule générale III 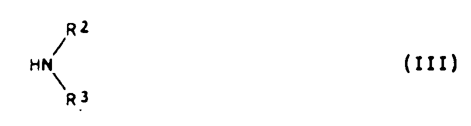 formules dans lesquelles R¹, R² et R³ possèdent les significations qui leur ont été attribuées ci-dessus, caractérisé en ce que l'on entreprend la réaction en présence d'anhydride carbonique à des températures de 10 à 150°C et des pressions de 0,5 à 50 bars.

2. Procédé suivant la revendication 1, caractérisé en ce que l'on utilise l'imine II et l'amine III dans le rapport molaire de 1:1 à 0,1:1.

3. Procédé suivant la revendication 1, caractérisé en ce que l'on utilise l'imine II et l'amine III dans le rapport molaire de 1:1 à 0,25:1.

4. Procédé suivant la revendication 1, caractérisé en ce que l'on utilise de 0,1 à 2 équivalents molaires d'anhydride carbonique par rapport à l'imine mise en oeuvre.

5. Procédé suivant la revendication 1, caractérisé en ce que l'on entreprend la réaction à une pression qui varie de 1 à 10 bars.
